# EUROPEAN PATENT SPECIFICATION

(11) **EP 2 323 573 B1**
(45) Date of publication and mention of the grant of the patent: **18.01.2012**
(21) Application number: 09792704.0
(22) Date of filing: 18.09.2009
(51) Int. Cl.: A61B 17/70, A61B 17/80

(54) **ANTERIOR TRANSPEDICULAR SCREW-AND-PLATE SYSTEM**
ANTERIORES TRANSPEDIKULÄRES SCHRAUBEN-PLATTEN-SYSTEM
SYSTÈME VIS-PLAQUE TRANSPÉDICULAIRE ANTÉRIEUR

(30) Priority: 18.09.2008 US 9803 P
(43) Date of publication of application: 25.05.2011
(60) Divisional application: 11004993.9
(73) Proprietor: Synthes GmbH, 4436 Oberdorf (CH)
(72) Inventor: OVERES, Thomas, CH-4513 Langendorf (CH); ZURSCHMIEDE, Silas, CH-2540 Grenchen (CH); FRIGG, Robert, CH-2544 Bettlach (CH); LECHMANN, Beat, CH-2540 Grenchen (CH)
(74) Representative: Lusuardi, Werther
(86) International application number: PCT/US2009/057454
(87) International publication number: WO 2010/033786

(56) References cited:
- DE-U1-202007 001 585
- US-A1- 2007 299 448
- US-A1- 2008 039 847

## Description

The present invention relates to a bone fixation device according to the preamble of claim 1.

Multilevel cervical spinal procedures result in relatively large loads on anterior cervical screw and plate systems, particularly in cases of severe three-column subaxial cervical spinal injuries and multilevel plated reconstructions in osteoporotic bone. Supplemental posterior instrumentation is therefore recommended to increase primary construct rigidity and limit potential compromise of the screw and plate systems. The increasing number of successfully performed posterior cervical pedicle screw fixations have enabled more stable fixations, however, most cervical pathologies are located anteriorly and are preferably addressed by an anterior approach.

Additionally, the use of pedicle screw fixations in the vertebrae area are typically limited by the position of the screw because improperly placing the screw results in potential arterial or spinal cord damage.
From US 2007/299448 a bone fixation device is known based on the preamble of claim 1.

Thus there is a need for a bone plating system that combines the advantages of an anterior approach with the superior biomechanical characteristics of a cervical pedicle screw fixation and expands the translation of the bone plating system while maintaining the proper bone screw placement.
The present invention relates to a bone fixation device comprising the features of claim 1.
In one preferred embodiment, the first and second rotatable eccentric members further enable the first plate and second plate to experience horizontal translation with respect to one another.

According to one embodiment, the bone fixation assembly attains its maximum length along the central longitudinal axis of the bone assembly when the first aperture is in its furthest position along the central longitudinal axis from the at least one fastener hole of the upper plate and the second aperture is in its furthest position along the central longitudinal axis from the at least one fastener hole of the lower plate and attains its minimum length along the central longitudinal axis when the first aperture is in its closest position along the central longitudinal axis to the at least one fastener hole of the upper plate and the second aperture is in its closest position along the central longitudinal axis to the at least one fastener hole of the lower plate.
A further preferred embodiment is set forth in claim 5.

### BRIEF DESCRIPTION OF SEVERAL VIEWS OF THE DRAWINGS

The foregoing summary, as well as the following detailed description of preferred embodiments of the application, will be better understood when read in conjunction with the appended drawings. For the purposes of illustrating the device of the present application, there is shown in the drawings preferred embodiments. It should be understood, however, that the application is not limited to the precise arrangement, structures, features, embodiments, aspects, and instrumentalities shown, and the arrangements, structures, features, embodiments, aspects and instrumentalities shown may be used singularly or in combination with other arrangements, structures, features, embodiments, aspects and instrumentalities. In the drawings:

Fig. 1 illustrates a top perspective view of an assembled plate according to a first preferred embodiment of the present invention;

Fig. 2 illustrates a bottom perspective view of the assembled plate of Fig. 1;

Figs. 3A illustrates a cross-sectional view of the assembled plate of Fig. 1, taken generally perpendicular to a longitudinal axis of the plate and through a central screw;

Fig. 3B illustrates a magnified cross-sectional view taken adjacent the central screw of Fig. 3A;

Fig. 4A illustrates a top perspective view of the assembled plate of Fig. 1 with upper and lower plates slightly reshaped and resized;

Fig. 4B illustrates a top perspective view of the assembled plate of Fig. 4A in a partially expanded position;

Fig. 4C illustrates a top perspective view of the assembled plate of Fig. 4A in a fully expanded position;

Fig. 5A illustrates a top plan, partially exploded view of the assembled plate of Fig. 4A with upper and lower plates slightly reshaped and resized;

Fig. 5B illustrates a top plan, partially exploded view of the assembled plate of Fig. 4B with upper and lower plates slightly reshaped and resized;

Fig. 5C illustrates a top plan, partially exploded view of the assembled plate of Fig. 4C with upper and lower plates slightly reshaped and resized;

Fig. 6 illustrates a top perspective view of the plate of Fig. 1 mounted to an anterior portion of a spine;

### DETAILED DESCRIPTION OF CERTAIN EMBODIMENTS

Certain terminology is used in the following description for convenience only and is not limiting. The words "right", "left", "top" and "bottom" designate directions in the drawings to which reference is made. The words "inwardly" and "outwardly" refer to directions toward and away from, respectively, the geometric center of the facet interference screw and designated parts thereof. The words, "anterior", "posterior", "superior", "inferior", "lateral", "sagittal", "axial", "coronal" and related words and/or phrases designate preferred positions and orientations in the human body to which reference is made and are not meant to be limiting. The terminology includes the above-listed words, derivatives thereof and words of similar import.

Certain embodiments of the present invention will now be discussed with reference to the aforementioned figures, wherein like reference numerals refer to like components. Preferred embodiments of the present invention are directed to a cervical anterior transpedicular screw-and-plate system. However, the preferred embodiments of the screw-and-plate system are not limited to applications or mounting in the anterior spine and may be utilized in the lumbar spine or for mounting to other bones in the human body, as would be apparent to one having ordinary skill in the art.

The plates described herein may be used in spinal fusion procedures in which a damaged or diseased disc (or part of a disc) is removed from between a pair of vertebrae and a spinal fusion spacer is placed between the vertebrae. The plates are applied to an anterior portion of the affected vertebrae to span the affected disc space, and may be fixed to the vertebrae using bone screws as will be described in more detail below. The plate functions to maintain the vertebrae aligned during the initial period following fixation in which fusion of the spacer to the adjacent vertebrae occurs. The plate may also function to share some of the axial spinal load applied to the fusion spacer to prevent extreme subsidence of the spacer into the vertebral body, such as where the patient has poor bone quality. The plates may also act to prevent the spacer from being expelled from the disc space during the initial post-operative period.

The plates may be used for single level (i.e. one-disc) fusion procedures, although in second and third preferred embodiments, the plates are used in multiple-level (i.e. multiple discs) fusion procedures. Some embodiments may be used for corpectomy procedures, in which at least a portion of a vertebral body is removed. While the plates herein are described with reference and application to the spine, it will be appreciated that features of the plates and the plates may have other applications, and can be applied to other bones and/or parts of the skeleton.

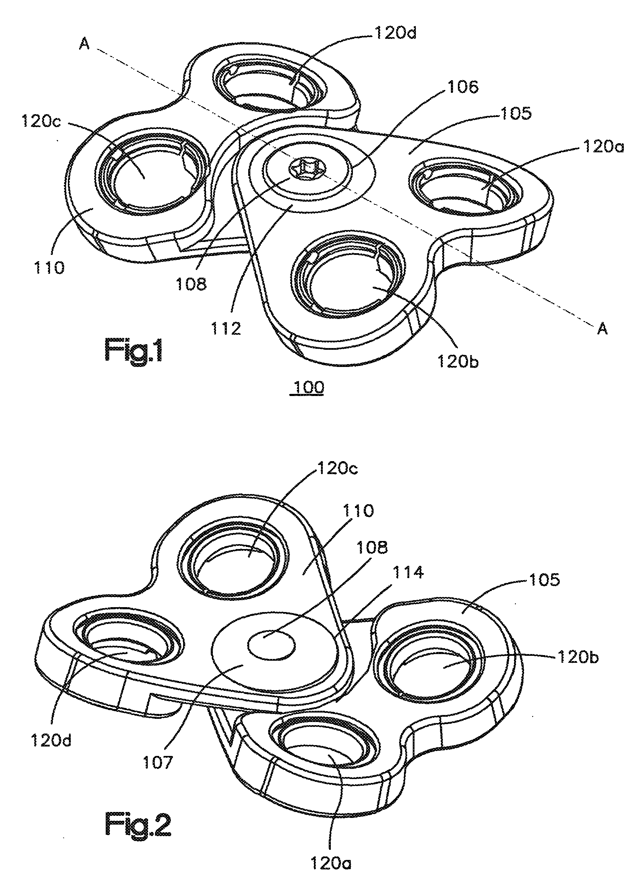

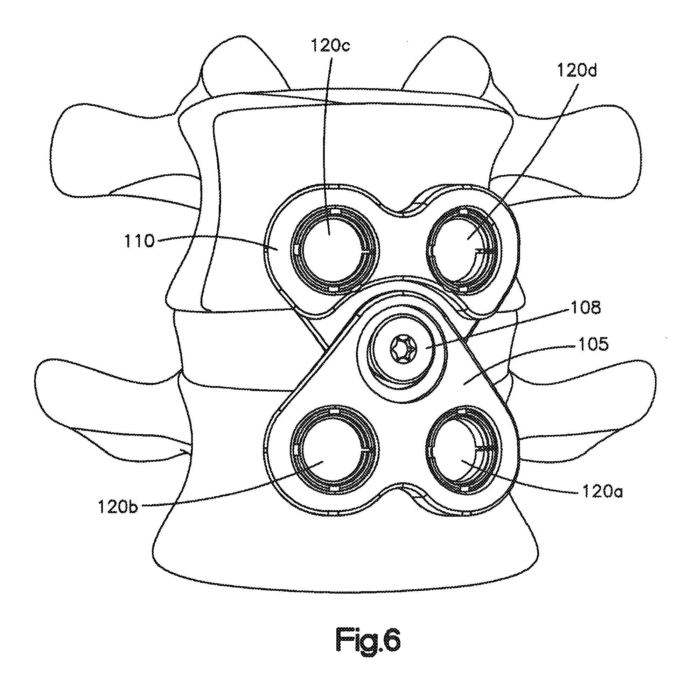

Referring to Figs. 1-6, a first embodiment of the system includes a locking plate 100, which has an upper plate 105, a lower plate 110, and a longitudinal axis A-A. The upper plate 105 has a rotatable, ring-shaped eccentric member 112, which contains an off-center aperture 106, and the lower plate 110 has a rotatable ring-shaped eccentric member 114 that contains an off-center aperture 107 for rotatably interconnecting the upper plate 105 to the lower plate 110 via a central screw 108. The first preferred embodiment of locking plate 100 includes two fastener holes 120a, 120b at an end of upper plate 105 and two fastener holes 120c and 120d at an end of lower plate 110. The fastener holes 120a, 120b, 120c, 120d may be configured to receive at least a portion of a bone fastener (See, for example, bone fastener 715 (Fig. 7B)), which may be inserted into a bone segment, such as a vertebral body (See Figs. 6 and 9). Although the plate 100 is shown with two pairs of fixation holes 120a, 120b, 120c, 120d, more than two pairs may be provided, for example so that plate 100 may span a greater length and thus be fastened to multiple locations along the spine or across multiple levels. Single holes (not shown), alternatively, may be provided as opposed to the pairs of fastener holes 120a, 120b, 120c, 120d. Additionally, each fastener hole 120a, 120b, 120c, 120d may contain a compression ring (not shown), as would be apparent to one having ordinary skill in the art, to receive the bone fasteners and the plate 100 may also have one or more visualization windows (not shown) extending from the upper surface of upper plate 105 through the lower surface of upper plate 105. The window may provide visual access to a disc space below the plate 100 when implanted into a patient's body.

Fig. 2 shows the under surface of the upper and lower plates 105, 110 in more detail. As can be seen in Fig. 2, the upper plate 105 is rotatably interconnected with the lower plate 110 via the insertion of the central screw 108 through both of the off-center apertures 106, 107 of the upper and lower plates 105, 110. Once the screw 108 is fully inserted through both of the off-center apertures 106, 107, the upper and lower plates 105 and 110 are secured to each other.

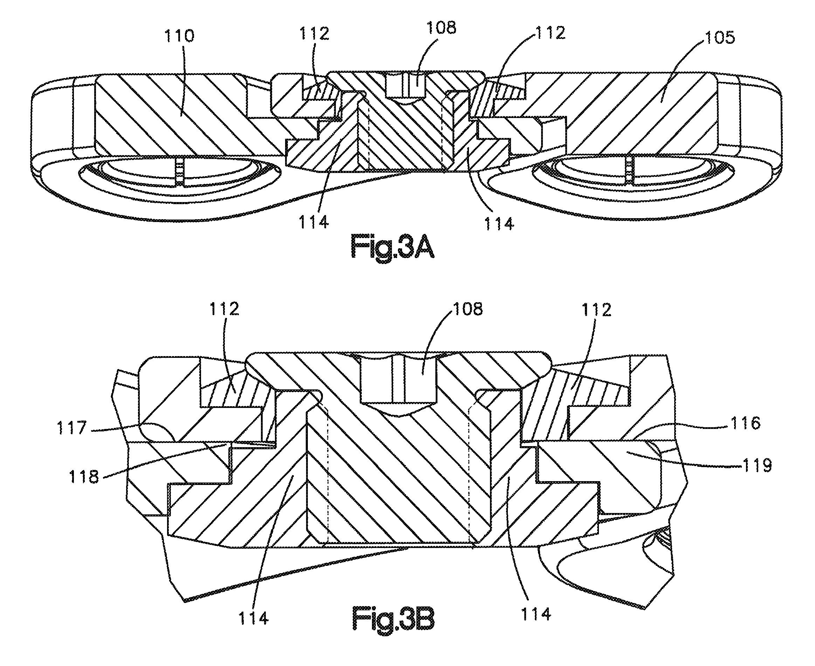

As can be seen in Figs. 3A and 3B, as the screw 108 is inserted through the off-center apertures 106, 107 and the screw 108 is tightened, the upper and lower plates 105, 110 are drawn closer together locking the position of the upper and lower plates 105, 110 relative to each other. Preferably, contact areas or surfaces 116, 117 of the upper plate 105 and contact areas or surfaces 118, 119 of lower plate 110 that contact each other in an assembled configuration may include a certain roughness, which allows the contact surfaces 116, 117, 118, 119 to exert friction relative to each other so that when the screw 108 is inserted though through the off-center apertures 106, 107 in the ring-shaped eccentric members 112, 114, the upper plate 105 and the lower plate 110 are secured to each other, thereby controlling the transverse sliding of the lower plate 105 with respect to the upper plate 110 along the longitudinal axis A-A of the plate 100.

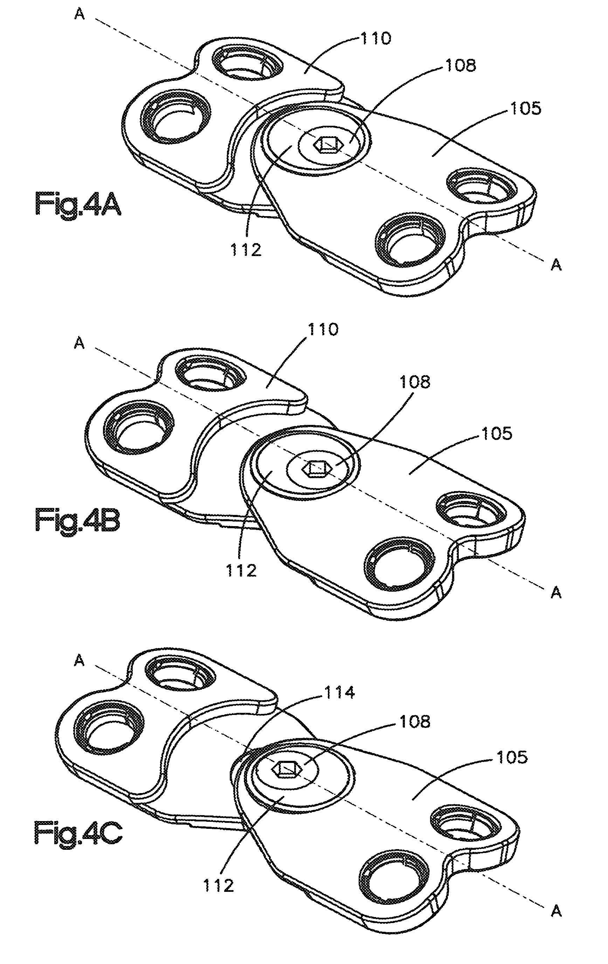

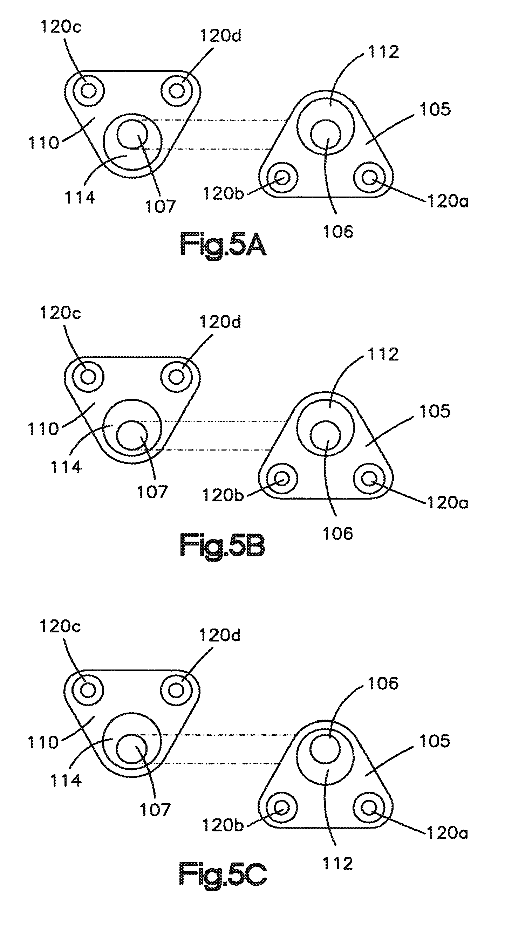

Each of the eccentric members 112, 114 are preferably rotatable relative to their respective upper and lower plates 105, 110. As can be seen in Figs. 4A and 5A, when the eccentric members 112, 114 are in their original positions or when the off-center aperture 106 of the upper plate 105 is in its closest position to a distal end of the upper plate 105 and the off-center aperture 107 of the lower plate 110 is in its closest position to a distal end of the lower plate 110, the screw plate 100 has its minimum length along the longitudinal axis A-A. In order to expand the length of the plate 100, the off-center eccentric members 112, 114 can be rotated individually or in concert to allow the upper and lower plates 105, 110 to translate relative to each other.

As can be seen in Figs. 4B and 5B, when the off-center eccentric member 114 of the lower plate 110 is rotated, thereby moving the off-center aperture 107 further away from the distal end of the lower plate 110, and closer to the distal end of the upper plate 105, the lower plate 110 is translated away from the upper plate 105 creating a greater length along the longitudinal axis A-A of the plate 100. Similarly, when the off-center eccentric member 112 of the upper plate 105 is rotated, thereby moving the off-center aperture 106 further away from the distal end of the upper plate 105 and closer to the distal end of the lower plate 110, the upper plate 105 is translated away from the lower plate 105 creating a greater length along the longitudinal axis A-A of the plate 100. Thus, as the off-center eccentric members 112, 114 are rotated in varying degrees, the length of the plate 100 along the longitudinal axis A-A is varied. As can be seen in Figs. 4C and 5C, when the eccentric member 114 of the lower plate 110 is rotated to allow the aperture 107 of the lower plate 110 to be in its furthest position along the longitudinal axis A-A relative to the distal end of the lower plate 110 and the off-center eccentric member 112 of the upper plate 105 is rotated to allow the aperture 106 of the upper plate 105 to be in its furthest position along the longitudinal axis A-A relative to the distal end of the upper plate 105, the plate 100 attains its greatest length along the longitudinal axis A-A.

Similarly, as will be appreciated by one of ordinary skill in the art, the upper and lower plates 105, 110 can attain varying horizontal translation relative to each other and the longitudinal axis A-A by rotating either or both of the eccentric members 112, 114 so that the apertures 106, 107 are positioned at various locations along a horizontal axis of plate 100, wherein the horizontal axis is generally perpendicular to the longitudinal axis A-A.

In conventional plating systems, a surgeon typically needs to rely on several different sized bone plates in order to account for the large number of possible dimensions of a patient's anatomy. In contrast, the use of the eccentric members 112, 114 of the plate 100 of the first preferred embodiment enables the use a limited number of different bone plates to account for the differing dimensions of a patient's anatomy. For instance, the below calculation illustrates how it is possible, through the rotation of the eccentric members 112, 114, to encompass a vast array of vertical distances with a limited number of plates. Where the eccentricity of the eccentric member 112 (e.g., in mm) of the upper plate 105 equals a distance between a center of the eccentric member 112 of the upper plate 105 and a center of the aperture 106 of the upper plate 105 and the eccentricity of the eccentric member 114 (e.g., in mm) of the lower plate 110 equals a distance between the center of eccentric member 112 of the lower plate 110 and a center of the aperture 107 of the lower plate 110, the maximal variability of the plate 100 along the longitudinal axis A-A would equal two times the eccentricity of the eccentric member 112 of the upper plate 105 plus two (2) times the eccentricity of the eccentric member 114 of the lower plate 110 ((2*eccentricity 112) + (2*eccentricity 114)). As mentioned above, the length of the plate 100 along the longitudinal axis A-A can be varied by rotating each or both of the eccentric members 112, 114 resulting in a single plate 100 that can be used for patients with varying anatomical dimensions.

One exemplary surgical technique, not forming part of the present invention, for implanting the plate 100 is described below, however, those skilled in the art will appreciate that the plate 100 utilizing numerous techniques and/or surgical steps that would be apparent to one having ordinary skill in the art, following a review of the present disclosure.

In use, for implantation of the plate 100 in the middle and lower cervical spine, an anterolateral approach is preferred. If the plate 100 is to be extended over several segments of the spine, a long incision is preferred. When exposing the vertebral bodies, the anterior longitudinal ligament is preferably removed or incised only in the areas where the intervertebral disc is to be bridged by the plate 100. Such a technique limits damage to the anterior longitudinal ligament in adjacent segments.

After the incision has been made, an image intensifier tool, such as Fluoroscopy (not shown), may be used to guide and monitor guide wires which are placed in the vertebra, preferably from the anterior side. Using spreaders, the cranial and caudal end-segments of the spine are spread and a corpectomy implant or natural bone is implanted to replace the removed segments. The distance between the guide wires is measured with, for example, a caliper instrument (not shown), which measurement can be utilized to determine plate size. Once the distance is determined, the plate 100 is preferably adjusted to the ideal length by rotating the eccentric members 112, 114 of the upper and lower plates 105, 110 and the adjusted plate 100 is placed over the guide wires. Once the proper size has been determined, before or after screws or fasteners have been inserted into the patient, the plate 100 is preferably locked by tightening the central screw 108.

To affix the plate 100 to the patient's vertebrae, cannulated screws are preferably guided over the guide wires and inserted through the fastener holes 120a, 120b, 120c, 120d. Although cannulated screws are preferably used, any heretofore known or hereafter developed means of fixation can be used. For example, cortical, pedicle or spongiosa screws may be placed into the vertebral body to fasten the plate 100 thereto. Additionally, once the fasteners or screws have been inserted, the screws can be locked using locking screws. An exemplary use of locking screws is disclosed in U.S. Patent No. 6,235,033, entitled "Bone Fixation Assembly".

## Claims

1. A bone fixation device (100) having a central longitudinal axis comprising:
a first plate (105) having an upper surface and a lower surface, at least one fastener hole (120a, 120b) configured to receive at least a portion of a first bone fastener, the fastener hole extending from the upper surface through to the lower surface,
and a second plate (110) having an upper surface and a lower surface, at least one fastener hole (120c, 120d) configured to receive at least a portion of a second bone fastener, the fastener hole extending from the upper surface through to the lower surface,
**characterised in that**
the first plate (105) further comprising a first rotatable eccentric member (112), the first eccentric member having a first aperture (106) for receiving at least a portion of a central fastener (108); and
the second plate (110) further comprising a second rotatable eccentric member (114), the second eccentric member having a second aperture (107) for receiving at least a portion of the central fastener,
wherein the first and second rotatable eccentric members enable the first plate and second plate to vertically translate with respect to one another, and the orientation of the first plate with respect to the second plate can be fixed by advancing the central fastener through the first and second apertures.

2. The bone fixation device of claim 1 wherein the first and second rotatable eccentric members further enable the first plate and second plate to experience horizontal translation with respect to one another.

3. The bone fixation device of claim 1 wherein the bone fixation assembly attains its maximum length along the central longitudinal axis when the first aperture is in its furthest position along the central longitudinal axis from the at least one fastener hole of the upper plate and the second aperture is in its furthest position along the central longitudinal axis from the at least one fastener hole of the lower plate.

4. The bone fixation device of claim 1 wherein the bone fixation assembly attains its minimum length along the central longitudinal axis when the first aperture is in its closes position along the central longitudinal axis to the at least one fastener hole of the upper plate and the second aperture is in its closest position along the central longitudinal axis to the at least one fastener hole of the lower plate.

5. The bone fixation device of claim 1 wherein at least a portion of the lower surface of the upper plate and at least a portion of the upper surface of the lower plate comprise a roughness to enable friction between said portions when the central fastener is inserted through the first and second apertures.

## Patentansprüche

1. Knochenfixationsvorrichtung (100) mit einer zentralen Längsachse umfassend:
eine erste Platte (105) mit einer Oberseite und einer Unterseite, und mindestens einem Befestigungsloch (120a, 120b), welches zur Aufnahme mindestens eines Abschnitts eines ersten Knochenfixationselements ausgebildet ist, wobei sich das Befestigungsloch von der Oberseite bis zur Unterseite erstreckt, und
eine zweite Platte (110) mit einer Oberseite und einer Unterseite, und mindestens einem Befestigungsloch (120c, 120d), welches zur Aufnahme mindestens eines Abschnitts eines zweiten Knochenfixationselements ausgebildet ist, wobei sich das Befestigungsloch von der Oberseite bis zur Unterseite erstreckt,
**dadurch gekennzeichnet, dass**
die erste Platte (105) zusätzlich ein erstes drehbares Exzenterteil (112) umfasst, wobei das erste Exzenterteil eine erste Öffnung (106) zur Aufnahme mindestens eines Teils eines zentralen Befestigungselements aufweist; und
die zweite Platte (110) zusätzlich ein zweites drehbares Exzenterteil (114) umfasst, wobei das zweite Exzenterteil eine zweite Öffnung (107) zur Aufnahme mindestens eines Teils des zentralen Befestigungselements aufweist, wobei
das erste und zweite drehbare Exzenterteil ermöglichen, dass sich die erste Platte und die zweite Platte relativ zueinander vertikal verschieben können, und wobei die Ausrichtung der ersten Platte relativ zur zweiten Platte durch Vorwärtsbewegen des zentralen Befestigungselements durch die erste und zweite Öffnung fixierbar ist.

2. Knochenfixationsvorrichtung nach Anspruch 1, **dadurch gekennzeichnet, dass** das erste und zweite drehbare Exzenterteil zusätzlich ermöglichen, dass die erste Platte und die zweite Platte eine horizontale Verschiebung relativ zueinander erfahren.

3. Knochenfixationsvorrichtung nach Anspruch 1, **dadurch gekennzeichnet, dass** die Knochenfixationsvorrichtung ihre maximale Länge entlang der zentralen Längsachse erreicht, wenn die erste Öffnung in ihrer entlang der zentralen Längsachse am weitesten von dem mindestens einen Befestigungsloch der oberen Platte entfernten Position ist und die zweite Öffnung in ihrer entlang der zentralen Längsachse am weitesten von dem mindestens einen Befestigungsloch der unteren Platte entfernten Position ist.

4. Knochenfixationsvorrichtung nach Anspruch 1, **dadurch gekennzeichnet, dass** die Knochenfixationsvorrichtung ihre minimale Länge entlang der zentralen Längsachse erreicht, wenn die erste Öffnung in ihrer entlang der zentralen Längsachse am nächsten bei dem mindestens einen Befestigungsloch der oberen Platte liegenden Position ist und die zweite Öffnung in ihrer entlang der zentralen Längsachse am nächsten bei dem mindestens einen Befestigungsloch der unteren Platte liegenden Position ist.

5. Knochenfixationsvorrichtung nach Anspruch 1, **dadurch gekennzeichnet, dass** mindestens ein Teil der Unterseite der oberen Platte und mindestens ein Teil der Oberseite der unteren Platte eine Rauhigkeit aufweisen, welche Reibung zwischen diesen Teilen ermöglicht, wenn das zentrale Befestigungselement durch die erste und zweite Öffnung eingeführt ist.

## Revendications

1. Système de fixation sur l'os (100) ayant un axe central longitudinal comportant :
une première plaque (105) présentant une surface supérieure et une surface inférieure, au moins un trou destiné à une pièce de fixation (120a, 120b) configuré afin de recevoir au moins une partie d'une première pièce de fixation sur l'os, le trou destiné à la pièce de fixation s'étendant de la surface supérieure jusqu'à la surface inférieure,
et une seconde plaque (110) présentant une surface supérieure et une surface inférieure, au moins un trou destiné à une pièce de fixation (120c, 120d) configuré pour recevoir au moins une partie d'un seconde pièce de fixation sur l'os, le trou destiné à la pièce de fixation sur l'os s'étendant de la surface supérieure jusqu'à la surface inférieure,
**caractérisé en ce que**
la première plaque (105) comporte, de plus, un premier élément excentré pouvant tourner (112), le premier élément excentré comportant une première ouverture (106) pour recevoir au moins une partie d'une pièce de fixation centrale (108) ; et
la seconde plaque (110) comprend, de plus, un second élément excentré pouvant tourner (114), le second élément excentré présentant une seconde ouverture (107) pour recevoir au moins une partie de la pièce de fixation centrale,
dans lequel les premier et second éléments excentrés pouvant tourner permettent à la première plaque et à la seconde plaque de translater verticalement l'une par rapport à l'autre, et dans lequel l'orientation de la première plaque par rapport à la seconde plaque peut être fixée en faisant avancer la pièce de fixation centrale à travers les première et seconde ouvertures.

2. Système de fixation sur l'os selon la revendication 1, dans lequel les premier et second éléments excentrés pouvant tourner permettent, de plus, à la première plaque et à la seconde plaque de subir une translation horizontale l'une par rapport à l'autre.

3. Système de fixation sur l'os selon la revendication 1 dans lequel l'ensemble de fixation sur l'os atteint sa longueur maximale le long de l'axe longitudinal central lorsque la première ouverture se trouve dans sa position la plus éloignée le long de l'axe longitudinal central du, au moins un, trou destiné à la pièce de fixation de la plaque supérieure et que la seconde ouverture se trouve dans sa position la plus éloignée le long de l'axe longitudinal central du, au moins un, trou destiné à la pièce de fixation de la plaque inférieure.

4. Système de fixation sur l'os selon la revendication 1 dans lequel l'ensemble de fixation sur l'os atteint sa longueur minimale le long de l'axe longitudinal central lorsque la première ouverture se trouve dans sa position la plus proche le long de l'axe longitudinal central du, au moins un, trou destiné à la pièce de fixation de la plaque supérieure et que la seconde ouverture se trouve dans sa position la plus proche le long de l'axe longitudinal central du, ou moins un, trou destiné à la pièce de fixation de la plaque inférieure.

5. Système de fixation sur l'os selon la revendication 1, dans lequel au moins une partie de la surface inférieure de la plaque supérieure et au moins une partie de la surface supérieure de la plaque inférieure comportent une zone de rugosité pour permettre une friction entre lesdites parties lorsque la pièce de fixation centrale est insérée à travers les première et seconde ouvertures.
